Europäisches Patentamt

European Patent Office

Office européen des brevets

(11) Publication number: **0 137 643**
**B1**

## (12) EUROPEAN PATENT SPECIFICATION

(45) Date of publication of patent specification: **09.08.89**

(51) Int. Cl.⁴: **A 61 L 15/00,** A 61 F 13/18

(21) Application number: **84305521.1**

(22) Date of filing: **14.08.84**

(54) Absorbent structure having a latex skin.

(30) Priority: **15.08.83 US 523244**
**25.06.84 US 624309**

(43) Date of publication of application:
**17.04.85 Bulletin 85/16**

(45) Publication of the grant of the patent:
**09.08.89 Bulletin 89/32**

(84) Designated Contracting States:
**AT BE CH DE IT LI LU NL SE**

(56) References cited:
**FR-A-2 293 914**
**GB-A-2 078 527**
**GB-A-2 087 730**
**GB-A-2 096 902**
**GB-A-2 115 702**

(73) Proprietor: **PERSONAL PRODUCTS COMPANY**
**Van Liew Avenue**
**Milltown New Jersey 08850 (US)**

(72) Inventor: **Holtman, Dennis C.**
**Box 125 RD Nr. 5, Old Clinton Road**
**Flemington New Jersey 08822 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

EP 0 137 643 B1

## Description

The present invention relates to an absorbent structure containing loosely compacted cellulosic fibers, which structure is particularly suitable for use in absorbent products requiring a substantial liquid holding capacity.

Absorbent structures such as disposable diapers, sanitary napkins and incontinence pads, are generally structured so as to have a facing sheet which is moisture-permeable, an absorbent batt which has a high liquid holding capacity, and a backing sheet which is moisture-impermeable. For any absorbent structure to be satisfactory, it is highly desirable for the structure to (1) readily accept liquid, (2) easily transport the liquid from one portion of the structure to another, (3) hold the liquid accepted, and (4) retain its original configuration even when wet. The facing sheet mentioned above must permit the liquid to penetrate the facing to reach the absorbent batt. The backing sheet keeps the liquid from leaking and, therefore, must be moisture-impermeable. The present invention relates to an improvement in the absorbent batt used for these absorbent structures.

Conventionally loosely compacted cellulosic fibrous batts are of substantially rectangular shape and are made by air-laying, for instance, wood pulp fibers or cotton linters, on a foraminous support. The batts may be laid in a prescribed shape or may be shaped subsequent to the air-laying of the fibers. These batts are incorporated by various techniques into products such as disposable diapers, sanitary napkins and incontinence pads.

Various techniques have been developed or suggested for reinforcing an absorbent batt to improve the retention of structural integrity. For instance, it has been suggested that scrim be used intermediate in the absorbent batt to improve the structural integrity. Other attempts are shown by US—A—3,017,304 who teaches forming a paper-like densified skin on one surface of the absorbent batt. The skin assists in transporting the liquid and lends integrity to the batt. In another instance, US—A—3,938,522 suggests densifying portions of the batt to both assist in transporting of liquid and strengthening the absorbent batt. US—A—4,233,345 sprays a solution of a coagulating material for a resin on one side of the batt. The resin is then dispersed on the pretreated surface providing rapid coagulation of the resin. Still other suggestions have involved wrapping the absorbent batt with tissue or anchoring the absorbent batt to the facing and/or the backing when the absorbent batt is used in a diaper-type product. Although these methods assisted in providing some integrity to absorbent structures, the absorbent structures still suffered from break up during shipping and use.

GB—A—2115702 discloses a disposable diaper comprising a vapour permeable, liquid impermeable backing sheet, an absorbent layer placed thereon and a liquid permeable sheet overlying the absorbent layer. The backing sheet is composed of a film produced by mixing a polyolefin resin, a filler and a liquid or waxy hydrocarbon polymer.

The present invention provides an absorbent structure which readily accepts liquid, easily transports the liquid from one portion of the structure to another, holds the liquid accepted and substantially retains its original configuration.

It has been discovered that a flexible absorbent batt having a high degree of integrity and contiguity can be made from loosely compacted cellulosic fibers, such as wood pulp fibers, which will readily accept and wick liquid while retaining a high liquid-holding capacity.

The present invention provides an absorbent structure comprising a stabilized absorbent batt, of loosely compacted cellulosic fibers having a thin latex skin formed on one surface thereof.

The new absorbent structure is made by air-laying cellulosic fibers to form a batt, applying a latex emulsion or suspension to at least a substantial portion of one surface of the batt and lightly pressing the batt.

Suitable latices include cross-linking latexes which crosslink at room temperature.

The present invention is now described, by way of example only, with reference to the accompanying drawings, in which:

Figure 1 is a perspective view of an absorbent structure of the present invention;

Figure 2 is a cross-sectional view of the absorbent structure of Figure 1 taken along line 2—2;

Figure 3 is a top view illustrating a particular configuration of another embodiment of the present invention;

Figure 4 is a top view illustrating still another particular configuration of an embodiment of the present invention; and

Figure 5 is a perspective view with certain portions broken away for clarity of illustration of one embodiment of a diaper of the present invention.

Tensile strength and structural integrity have always been desired in cellulosic fibrous batts. Fibrous batts are commonly formed of loosely compacted, cellulosic fibers which are primarily held together by interfiber bonds requiring no added adhesive. However, when using the absorbent batt, integrity is important to prevent separation and breakage of the absorbent batt.

The present invention, unlike the densified paper skin or thin gel skin of the above-mentioned U.S. patents, provides a thin layer or pattern of a latex film on one surface of the absorbent batt. The film retains its continuity providing contiguity and structural integrity to the absorbent batt. It is surprising to observe that the latex applied to one surface of the batt markedly improves the tensile strength without adversely affecting the liquid holding capacity or the wicking ability of the absorbent structure. Preferably, the absorbent batt has a dry tensile strength of at least 88.6 g/cm (225 g/inch). Furthermore, the structure is still very flexible and has superior wet strength.

Wet strength in an absorbent product like a disposable diaper is highly desirable so that on the initial wetting the batt does not break apart. It is theorized that the three-dimensional latex film may provide this high degree of tensile strength and wet strength without adversely affecting the other desirable properties of the absorbent batt. Preferably, the wet tensile strength of the absorbent batt is at least 19.7 g/cm (50 g/inch).

The absorbent structure of the present invention is a fibrous batt formed of loosely compacted cellulosic fibers. Preferably, the latex is a room temperature, crosslinking latex which is applied as a thin layer or in a continuous pattern as desired on one surface of the cellulosic fibrous batt. The batt is then subjected to slight compression so that the latex comes in contact with the fibers on the surface so treated.

Although the density of the absorbent batt increases in the latex region, penetration of the latex is minimal so that only the immediate surface area is affected.

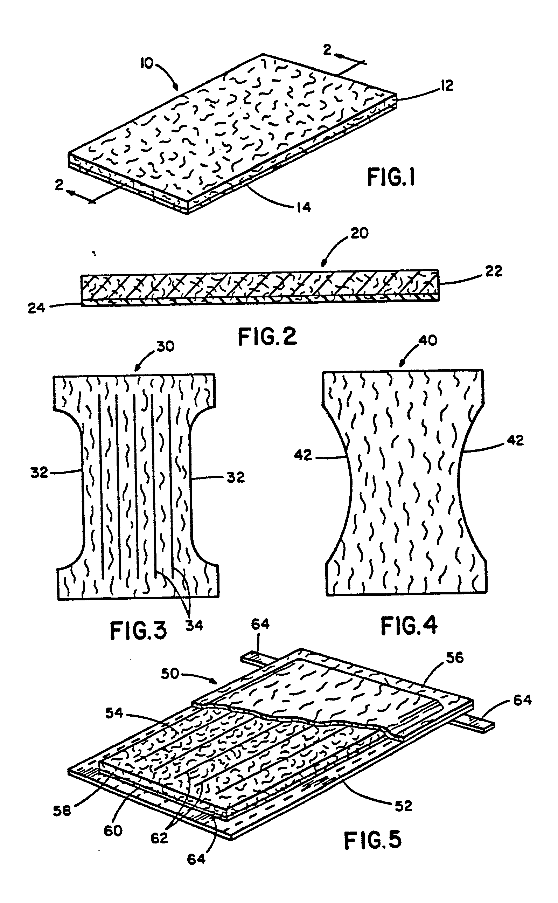

In Figure 1, an absorbent batt 10 is provided. The latex surface 14 represents a very small portion of the overall depth of the absorbent batt 10. The remainder of the batt 12 remains in loosely compacted form. Figure 2 is a cross-sectional view taken along lines 2—2 of Figure 1 providing an absorbent batt 20 having a fluffed, loosely compacted fibrous region 22 and a thin region of latex 24.

Figure 3 illustrates a top view of a typical configuration of the absorbent structure of the present invention. The absorbent batt 30 is reduced in width at the central portion 32 to provide a shaped, absorbent batt. To lend further to the integrity of the batt, embossed lines 34 are provided. The embossed lines 34 will assist in enhancing the wicking of liquid from one region to another of the absorbent batt 30.

Figure 4 illustrates still another shaped absorbent batt 40 wherein the central portion 42 is diminished in size to provide an hour-glass shape.

Figure 5 illustrates a typical disposable diaper structure 50 using the absorbent batt 54 of the present invention. The diaper 50 comprises a moisture-pervious facing 56 and a moisture-impermeable backing 52 with the absorbent batt 54 held in place between the backing 52 and the facing 56. The absorbent batt has a fluffed loosely compacted portion 58 and a thin densified portion 60 having been treated with latex. Embossing lines 62 are provided to enhance the wicking in the diaper structure. The batt 54 is held in place and the facing 56 and backing 52 are laminated by glue lines 66. Tape tabs 64 are provided for securing the diaper about the waist of the infant.

The absorbent batts of the present invention are prepared by forming a substantially uniform batt of air-laid loosely compacted cellulosic fibers. The fibers generally are wood pulp fibers which have been subjected to conventional chemical processing. Typically, chemically processed wood pulp fibers appear in ribbon-like form and, upon air-laying, become entangled to provide a degree of cohesiveness in fibrous web integrity.

Non-delignified wood pulp fibers are also suitable and may have an additive present to enhance the cohesiveness of the fibers to form a reasonably stable absorbent batt.

In accordance with the present invention, whether the batt is made from chemically processed fibers or non-delignified wood pulp fibers, the batt is provided with one surface area effectively treated with a thin layher of latex film. The layer may provide a continuous film or may be applied in a predetermined continuous pattern. At least 35 percent of the surface is treated with the thin layer of latex film and preferably the entire surface area is so treated. The layer is generally located on the planar surface away from the point of liquid entry when the product is in use.

The air-laid, loosely compacted absorbent batt is subjected to a small degree of compaction so that the latex emulsion applied to the surface penetrates the surface slightly. Suitable latexes include the latexes which crosslink at room temperature. Typical of such latexes are those mentioned in US—A—3,678,098 to Lewis et al.

The latex is applied after the loosely compacted cellulosic fibrous batt is formed. However, the final shape of the batt need not be determined at this point as further cutting or shaping of the batt may be made at a later time. The latex at room temperature is applied to one surface of the batt in either a continuous form or in a continuous predetermined pattern. The latex should cover at least 35 percent of the surface, but preferably 90 percent or more of the surface. In one embodiment, the latex skin is discontinuous and covers at least 35 percent of the surface. After it is applied, the film forms at room temperature and the film increases in molecular weight building toward water insolubility and the thin, stabilized area of the batt is formed.

The absorbent batts of the present invention are particularly useful in disposable products such as disposable diapers, sanitary napkins and incontinence pads. Each of the disposable products usually contains a moisture-impermeable backing sheet, an absorbent batt in superposed position and a moisture-permeable facing or cover in superposed position on the side of the batt opposite the backing sheet.

The disposable product, when ideally constructed, permits a void of liquid onto the facing to be rapidly drawn through the facing into the absorbent batt for storage. Leakage, when the batt becomes substantially wetted, is prevented by the backing sheet. However, if the absorbent batt has lost its integrity and in fact has separations present or breaks, there is a loss of ability to transport liquid to the extremities of the batt, thus liquid will have a tendency to leak out the side edges of the disposable product. Therefore, the provision of the batt with high tensile strength in accordance with the present invention, provides a suitable absorbent structure to place in such a disposable product. One advantage is that when the absorbent structure becomes wet, the film still retains the contiguity of the overall structure and prevents break up. Also, because the product retains its shape, sagging and destruction of the product's aesthetics is avoided.

The following example illustrates a specific embodiment of the present invention and is not limiting in any way.

Example

A batt of chemically delignified wood pulp fibers is formed by using a hammer mill and light compression. The batt is rectangular, about 1.27 cm ($\frac{1}{2}$ inch) thick and weighs about 52 grams. A paper-like densified skin is formed on one side (see US—A—3,017,304) and five embossed lines running most of the length of the batt are placed substantially equidistant from one another. The batt just described is identified as "The Control Batt". Sample A is a batt having the characteristics of The Control Batt except that on the surface possessing the paper-like densified skin, a latex emulsion is applied in the amount of 1.75 g by spraying over the batt surface. The sprayed emulsion is a six percent latex emulsion solution identified as E—1179N produced and sold by Rohm and Haas Corporation. The batts are tested for dry density, liquid-holding capacity, wet and dry tensile strength and batt stability. Results of the test appear in Table I below:

TABLE I

| | | | Tensile Strength | | Batt Stability | |
| Sample | Dry Density g/cc | Liquid Holding Capacity ml/g | Dry g/cm (g/in) | Wet g/cm (g/in) | Exposed Film (%) | Weight Loss (gm) |
|---|---|---|---|---|---|---|
| Control | .102 | 5.9 | 50.4 (128) | 12.6 (32) | 14.0 | 18.0 |
| Sample A | .103 | 5.9 | 126.4 (321) | 22.4 (57) | 0.3 | 2.5 |

It should be noted that the liquid holding capacity and the density of the batts are substantially equal. However, the tensile strength, both wet and dry, are substantially improved when a thin-coating latex is used on the absorbent batt described above. Thus, the objects of the invention are accomplished without loss of the liquid-holding capacity or markedly affecting the product density.

## Claims

1. An absorbent structure comprising a stabilised absorbent batt (10), said batt (10) being comprised of loosely-compacted cellulosic fibers having a thin latex skin (14) formed on one surface thereof.

2. The absorbent structure of Claim 1 wherein said latex skin (14) is continuous and covers at least 90 percent of one surface of the batt.

3. The absorbent structure of Claim 1 wherein said latex skin (14) is discontinuous and covers at least 35 percent of one surface of the batt.

4. The absorbent structure in any one of Claims 1 to 3 wherein the dry tensile strength of said absorbent batt is at least 88.6 g/cm (225 g/in).

5. The absorbent structure of Claim 1 wherein the wet tensile strength of said absorbent batt is at least 19.7 g/cm (50 g/in).

6. A disposable absorbent product having a moisture-pervious facing sheet (56) defining an inside surface for direction toward the wearer, a moisture-impervious sheet (52) substantially coextensive with said facing sheet (56) and defining an outside surface, and an absorbent batt (54) according to any one of Claims 1 to 5 positioned between said facing sheet (56) and said backing sheet (52) with the thin latex skin (60) in contact with said backing sheet (52).

7. The disposable product of Claim 6 which is in the form of a diaper.

8. The disposable product of Claim 7 which is in the form of a sanitary napkin.

## Patentansprüche

1. Eine absorbierende Struktur, bestehend aus einem stabilisierten absorbierenden Vlies (10), wobei das genannte Vlies (10) aus lose kompaktierten Zellulosefasern mit einer dünnen Latexhaut (14) besteht, die auf einer Oberfläche derselben gebildet ist.

2. Die absorbierende Struktur nach Anspruch 1, bei der die genannte Latexhaut (14) kontinuierlich ist und mindestens 90% einer Oberfläche des Vlieses bedeckt.

3. Die absorbierende Struktur nach Anspruch 1, bei der die genannte Latexhaut (14) diskontinuierlich ist und mindestens 35% einer Oberfläche des Vlieses bedeckt.

4. Die absorbierende Struktur nach einem der Ansprüche 1 bis 3, bei der die trockene Zugfestigkeit des genannten absorbierenden Vlieses mindestens 88,6 g/cm (225 g/in) beträgt.

5. Die absorbierende Struktur nach Anspruch 1, bei der die nasse Zugfestigkeit des absorbierenden Vlieses mindestens 19,7 g/cm (50 g/in) beträgt.

6. Ein wegwerfbares absorbierendes Erzeugnis mit einer feuchtigkeitsdurchlässigen Deckschicht (56), welche eine Innenseite bildet, die dem Träger zugewandt ist, mit einer feuchtigkeitundurchlässigen Schicht (52), die sich im wesentlichen so weit wie die Deckschicht (56) erstreckt und eine Außenseite bildet, und einem absorbierenden Vlies (54), nach einem der Ansprüche 1 bis 5, das zwischen der genannten Deckschicht (56) und der genannten Rückschicht (52) positioniert ist, wobei die dünne Latexhaut (60) mit der Rückschicht (52) in Berührung steht.

7. Das wegwerfbare Erzeugnis nach Anspruch 6, welches in Form einer Windel vorliegt.

8. Das wegwerfbare Erzeugnis nach Anspruch 7, welches in Form einer hygienischen Binde vorliegt.

**Revendications**

1. Structure absorbante comprenant un voile absorbant stabilisé (10), ce voile (10) étant formé de fibres cellulosiques lâchement serrées comportant une peau de latex mince (14) formée sur une de ses surfaces.

2. Structure absorbante suivant la revendication 1, dans laquelle la peau de latex (14) est continue et couvre au moins 90% d'une surface du voile.

3. Structure absorbante suivant la revendication 1, dans laquelle la peau de latex (14) est discontinue et couvre au moins 35% d'une surface du voile.

4. Structure absorbante suivant l'une quelconque des revendications 1 à 3, dans laquelle la résistance à la traction à l'état sec du voile absorbant est d'au moins 88,6 g par cm (225 g par pouce).

5. Structure absorbante suivant la revendication 1, dans laquelle la résistance à la traction à l'état mouillé du voile absorbant est d'au moins 19,7 g par cm (50 g par pouce).

6. Produit absorbant jetable comportant une feuille de parement perméable à l'humidité (56) définissant une surface interne à diriger vers l'utilisateur, une feuille imperméable à l'humidité (52) en substance coextensive avec la feuille de parement (56) et définissant une surface extérieure, et un voile absorbant (54) suivant l'une quelconque des revendications 1 à 5 positionné entre la feuille de parement (56) et la feuille de support (52), la peau de latex mince (60) étant en contact avec la feuille de support (52).

7. Produit jetable suivant la revendication 6, qui a la forme d'une couche.

8. Produit jetable suivant la revendication 7, qui a la forme d'une serviette hygiénique.

FIG.1

FIG.2

FIG.3

FIG.4

FIG.5